# EUROPEAN PATENT SPECIFICATION

(11) **EP 0 626 818 B1**
(45) Date of publication and mention of the grant of the patent: **15.05.2002**
(21) Application number: 93903477.3
(22) Date of filing: 14.01.1993
(51) Int. Cl.: A61B 1/00

(54) **MEDICAL CATHETER**
MEDIZINISCHES KATHETER
CATHETER MEDICAL

(30) Priority: 22.01.1992 US 824023
(43) Date of publication of application: 07.12.1994
(73) Proprietor: WINSTON, Thomas R., Leawood, KS 66211 (US)
(72) Inventor: WINSTON, Thomas, R., Leawood, KS 66211 (US); NEET, John, M., Shawnee, KS 66217 (US)
(74) Representative: VOSSIUS & PARTNER
(86) International application number: US9300314
(87) International publication number: WO9314689

(56) References cited:
- WO-A-92/16140
- US-A- 4 576 177
- US-A- 4 587 972
- US-A- 4 869 258
- US-A- 4 967 745

## Description

This invention relates generally to the medical catheters and in one aspect to an ultrasonic transducer system having transducers located externally of the catheter for sensing the character and configuration of the area that is to be medically treated by the catheter.

It has long been known that cardiovascular problems are caused by the presence of atherosclerotic plague on the walls of veins and arteries, especially coronary arteries. For some time, there has been interest in the use of laser energy to remove plaque and to treat abnormalities on internal organs in the body. Typically, it is proposed to use a catheter that contains optical fibers for transmitting the laser energy through the catheter to the area that is to undergo treatment. Lasers have also been used to treat other medical problems such as tumors or other abnormalities in the colon, esophagus, prostate and other areas of the body.

U.S. Patent No. 4,576,177 to Webster discloses a catheter which includes optical fibers in combination with an ultrasonic transducer having the capability of transmitting and receiving ultrasonic signals. The reflections of the ultrasonic signals from the tissue are received by the transducer to provide information as to the character and configuration of the tissues so that the laser energy can be applied properly to the plaque lesion and not to unoccluded artery walls or other healthy tissues.

In the device disclosed in the Webster patent, the ultrasonic transducer takes the form of a flat ring which is oriented at an angle to the axis of the catheter. With this arrangement, it is necessary to rotate the catheter through a full 360° circle in order to direct the ultrasound at the entire circumference of the artery. The need to manually rotate the catheter is at best a severe inconvenience and an inaccurate procedure because the catheter cannot be accurately stepped through incremental arcs in order to provide a reliable profile of the arterial plaque. If the ultrasound techniques are inadequate to provide the instrument with an accurate configuration of the plaque, the laser energy can be misdirected such that it is not only ineffective in treating the problem but also possibly destructive of healthy tissues.

The transducer is located in the tip of the catheter, and the space that it requires there causes the catheter to have a relatively large tip. It is also necessary to extend electrical wires through the catheter to the transducer, and this complicates the catheter construction and adds to its cost.

US-A-4 967 745 discloses a molded fiber optic plug for retaining optical fibers in a fixed position within a laser catheter in which an optically polished tubular moldable plug body is used to secure a plurality of optical fibers within a laser catheter. The laser catheter may be used in combination with a guide catheter. In the laser catheter control wires are used to position the distal end of the catheter.

The present invention is defined by the features of the claims and is an improvement over the catheters described in the prior art.

In accordance with the invention, a catheter equipped with optical fibers for transmitting laser energy or another medical treatment scheme such as an abrasive catheter head is also provided with an ultrasonic sensing system that is able to accurately determine the character and configuration of the entirety of an artery wall or other treatment area without the need for manual turning of the catheter. Preferably the ultrasound system is located outside of the catheter and makes use of optical fibers to transmit ultrasonic signals from the transducers to the catheter tip and return echoes to the transducers. As a result, the catheter tip is not occupied by transducers and can be reduced in size. At the same time, the overall complexity and cost of the catheter are reduced.

In different embodiments of the invention, optical fibers used for the transmission of ultrasonic signals. are arranged in different patterns relative to optical fibers used to transmit laser energy for photoablative treatment of plaque or other tissue abnormalities. In one form of the invention, there is a single fiber carrying ultrasonic signals arranged inside a rotary probe. The fiber can be reciprocated along a radius of the probe to provide coverage of the entire area ahead of the probe with forward looking ultrasound. In another form, the fibers are arranged in a radial row to sweep the entire area ahead of the catheter when the probe is rotated.

The catheter may comprise a plurality of disks extending radially from the probe. The disks carry circumferentially spaced ultrasonic transducers which connect with fibers extending through the probe to its tip end. Electrical power is supplied to the transducers through slip ring electrical contacts. In this manner, a large number of ultrasonic transducers can be used to provide large amounts of information concerning the configuration and location of the plaque deposit or other affliction that is to be treated.

Other and further objects of the invention together with the features of novelty appurtenant thereto, will appear in the course of the following description.

In the accompanying drawings which form a part of the specification and are to be read in conjunction therewith and in which like reference numerals are used to indicate like parts in the various views:
Fig. 1 is a fragmentary side elevational view of a catheter constructed according to one embodiment of the present invention, with portions shown-in section for illustrative purposes and the break lines indicating continuous length;
Fig. 2 is a fragmentary sectional view on an enlarged scale taken generally along line 2-2 of Fig. 1 in the direction of the arrows;
Fig. 3 is a fragmentary sectional view of the outer end portion of the probe for the catheter, showing a control wire and actuator for adjusting the radial position of an optical fiber used to transmit ultrasonic signals along the catheter;
Fig. 4 is a fragmentary sectional view on an enlarged scale showing a tapered transition element between an ultrasonic transducer and the optical fiber which transmits ultrasonic signals;
Fig. 5 is a fragmentary sectional view similar to Fig. 4, but showing an alternative transition element.

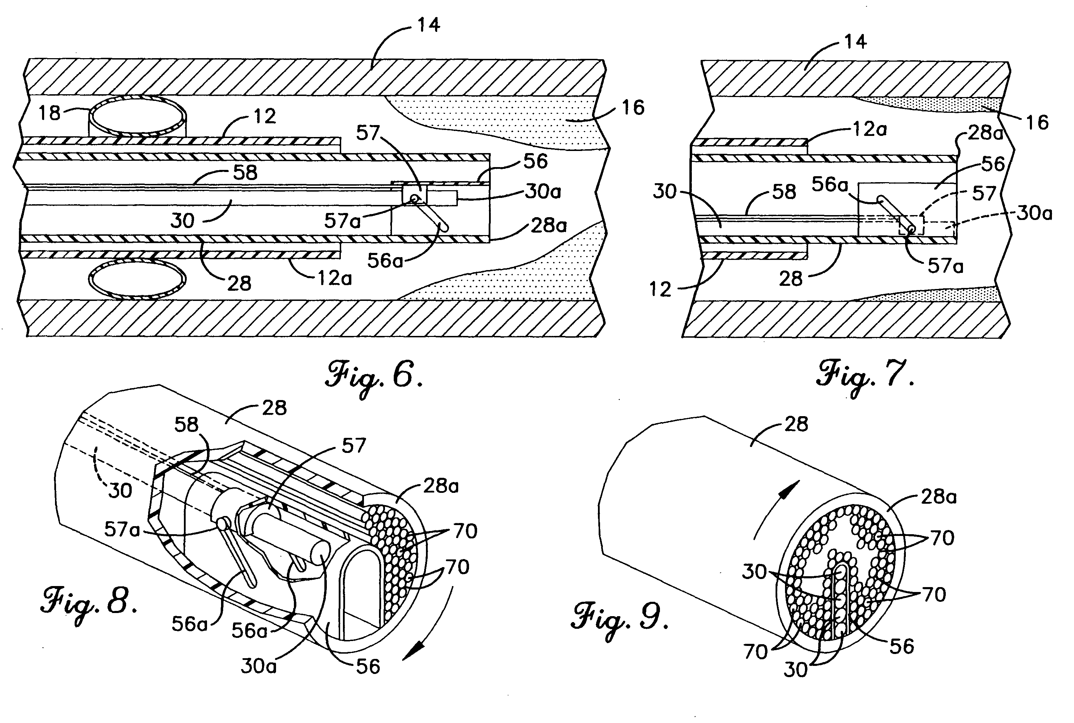

The embodiments of Figures 6 - 15 do not fall within the definition of the claimed invention but are usefue for its understanding.
Fig. 6 is a fragmentary sectional view on an enlarged scale of the tip of the catheter, with the optical fiber located near the center of the probe;
Fig. 7 is a fragmentary sectional view on an enlarged scale similar to Fig. 6 but showing the fiber adjusted to a location near the circumference of the probe;
Fig. 8 is a fragmentary perspective view of the tip of the probe, with portions broken away for clarity and the optical fiber located near the center of the probe;
Fig. 9 is a fragmentary perspective view similar to Fig. 8 but showing an alternative embodiment in which the probe contains plural optical fibers arranged in a radial row;

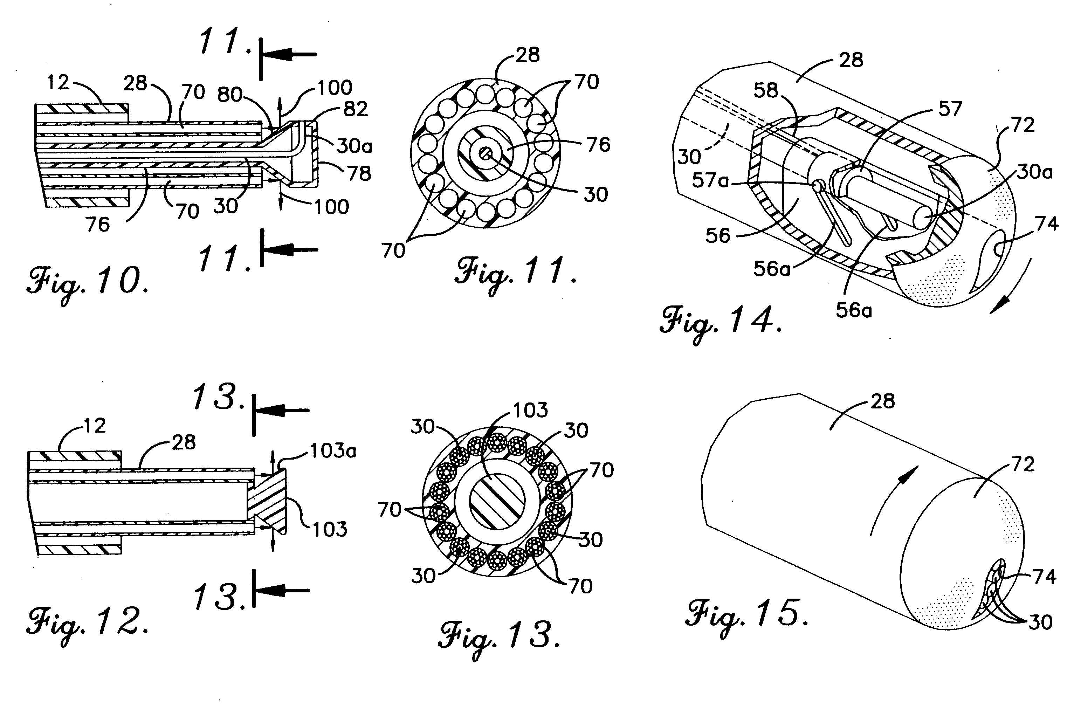

Fig. 10 is a fragmentary side elevational view of the tip of a catheter constructed according to another embodiment;
Fig. 11 is a fragmentary sectional view on an enlarged scale taken generally along line 11-11 of Fig. 10 in the direction of the arrows;
Fig. 12 is a fragmentary sectional view of the tip of a catheter constructed according to another alternative;
Fig. 13 is a fragmentary sectional view on an enlarged scale taken generally along line 13-13 of Fig. 12 in the direction of the arrows;
Fig. 14 is a fragmentary perspective view on an enlarged scale of the tip of a catheter constructed according to yet another embodiment, with portions broken away for illustrative purposes;
Fig. 15 is a fragmentary perspective view similar to Fig. 14 but showing the tip end of yet another alternative catheter;

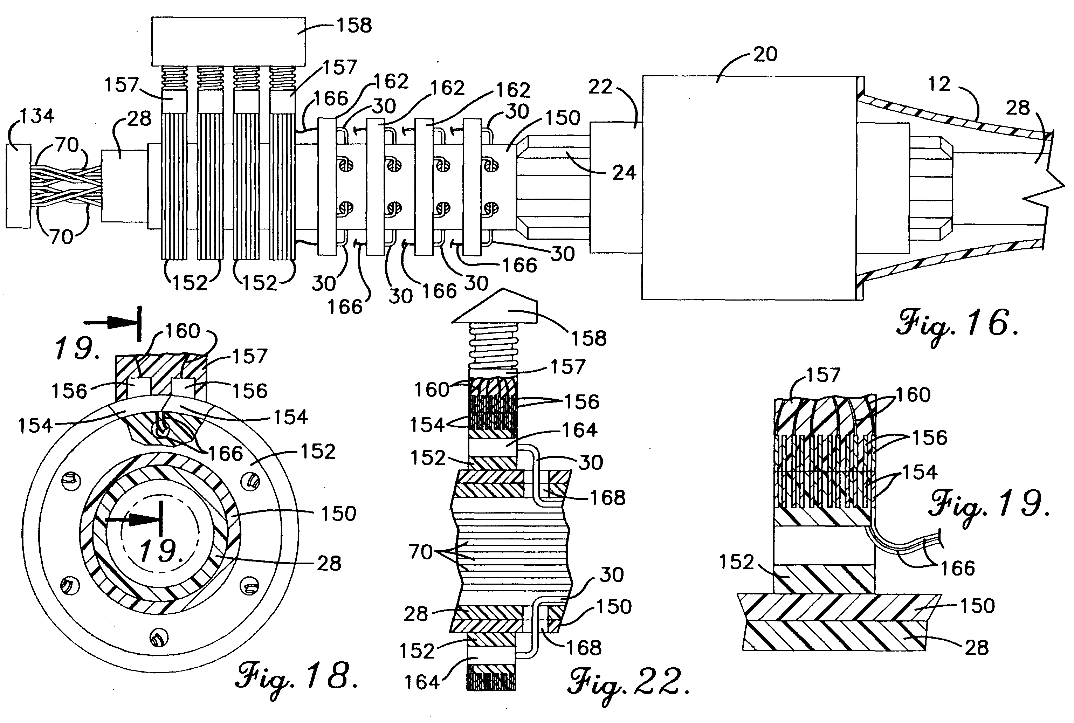

Fig. 16 is a fragmentary side elevational view of a catheter constructed according to another embodiment of the invention;
Fig. 17 is a fragmentary elevational view on an enlarged scale showing the external end portion of the catheter depicted in Fig. 16;
Fig. 18 is a fragmentary sectional view on an enlarged scale taken generally along line 18-18 of Fig. 17 in the direction of the arrows;
Fig. 19 is a fragmentary sectional view on an enlarged scale taken generally along line 19-19 of Fig. 18 in the direction of the arrows;

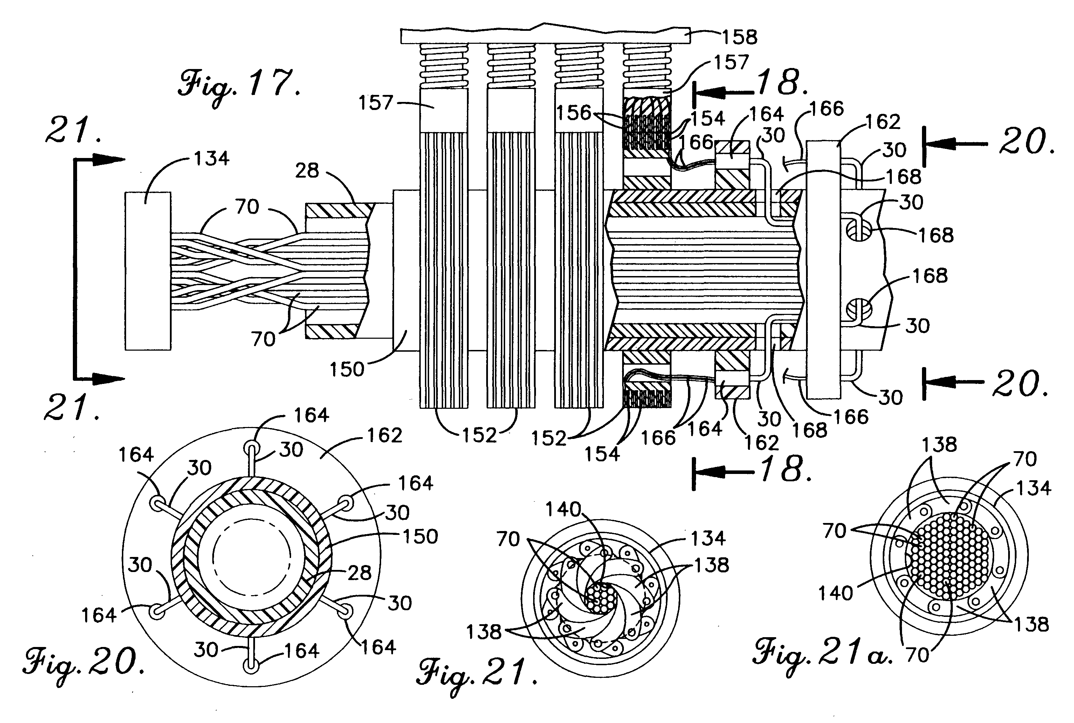

Fig. 20 is a fragmentary sectional view taken generally along line 20-20 of Fig. 17 in the direction of the arrows;
Fig. 21 is a fragmentary end elevational view taken generally along line 21-21 of Fig. 17 in the direction of the arrows, with the shutter mechanism partially closed;
Fig. 21a is a fragmentary end elevational view similar to Fig. 21 but with the shutter mechanism fully open;

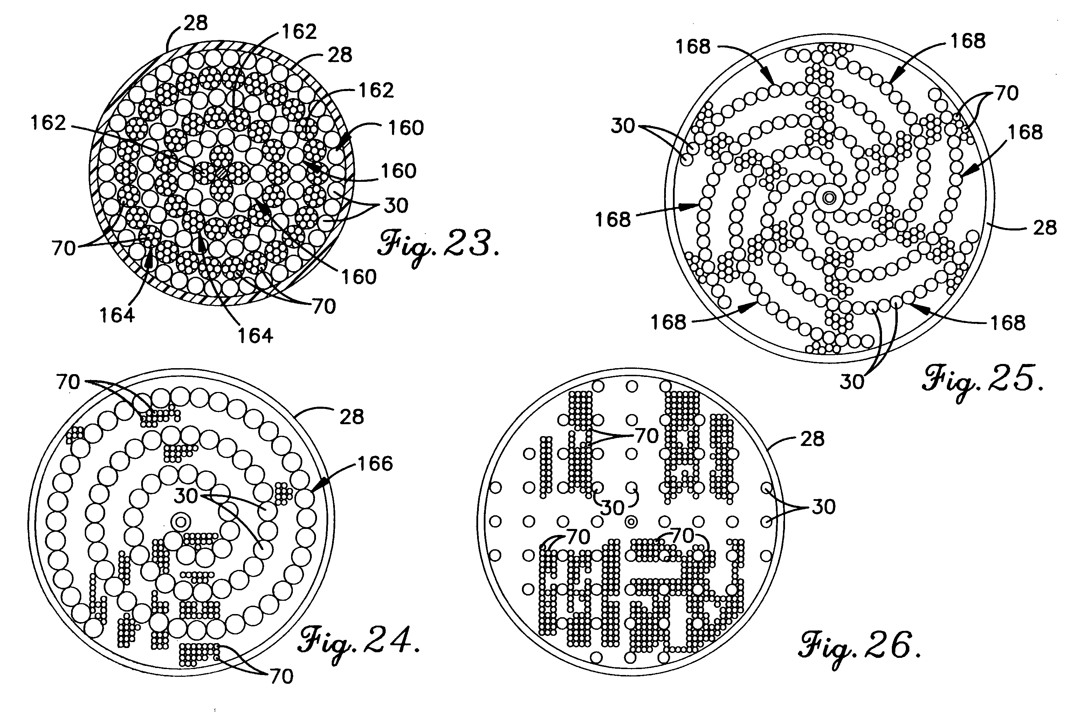

Fig. 22 is a fragmentary sectional view showing a modification of the catheter depicted in Fig. 16;
Figs. 23-26 are sectional views depicting different patterns in which optical fibers carrying ultrasonic signals and optical fibers carrying laser energy can be arranged in the catheter in accordance with the present invention.

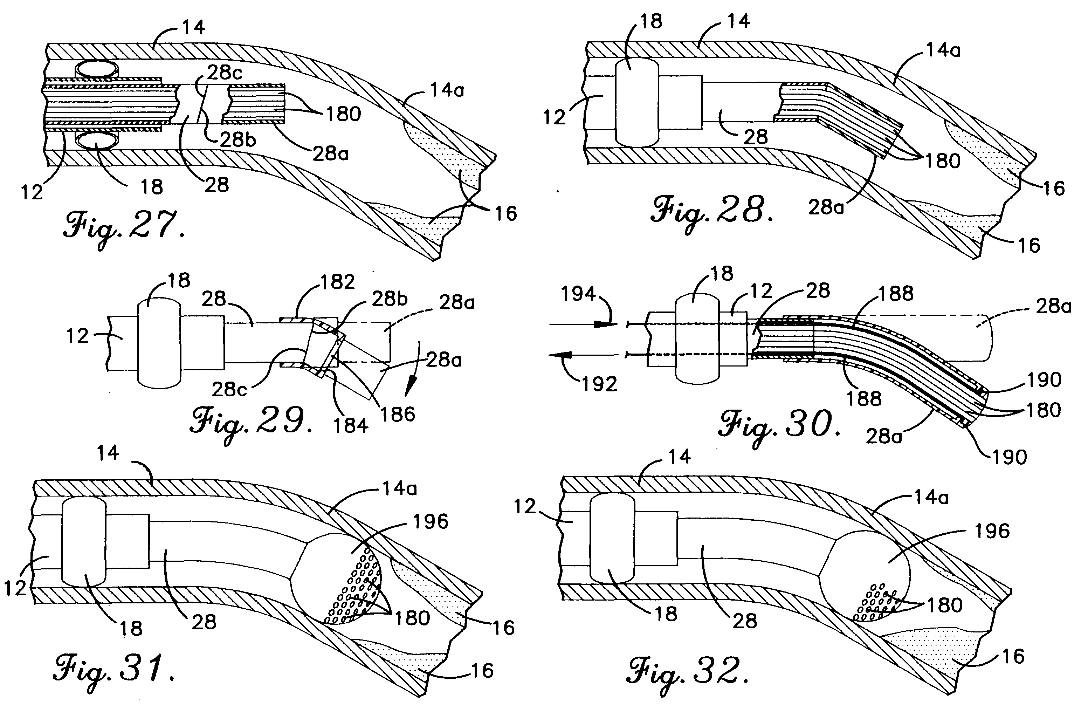

The embodiments of Figures 27 - 29, 31 and 32 do not fall within the definition of the claimed invention but are useful for its understanding.
Fig. 27 is a fragmentary sectional view of an alternative embodiment of a catheter inserted in an artery;
Fig. 28 is a fragmentary sectional view similar to Fig. 27, but with the tip of the probe of the catheter rotated to accommodate a bend in the artery;
Fig. 29 is a fragmentary elevational view of another alternative form of the catheter depicting rotation of the probe tip to accommodate bends in the arterial configuration;
Fig. 30 is a fragmentary sectional view of a catheter embodying the present invention and depicting the manner in which the probe tip can be bent to accommodate bends in the arterial configuration;
Fig. 31 is a fragmentary sectional view of still another catheter inserted in an artery having a bend; and
Fig. 32 is a fragmentary sectional view of a further embodiment of a catheter, with the catheter inserted in an artery having a bend.

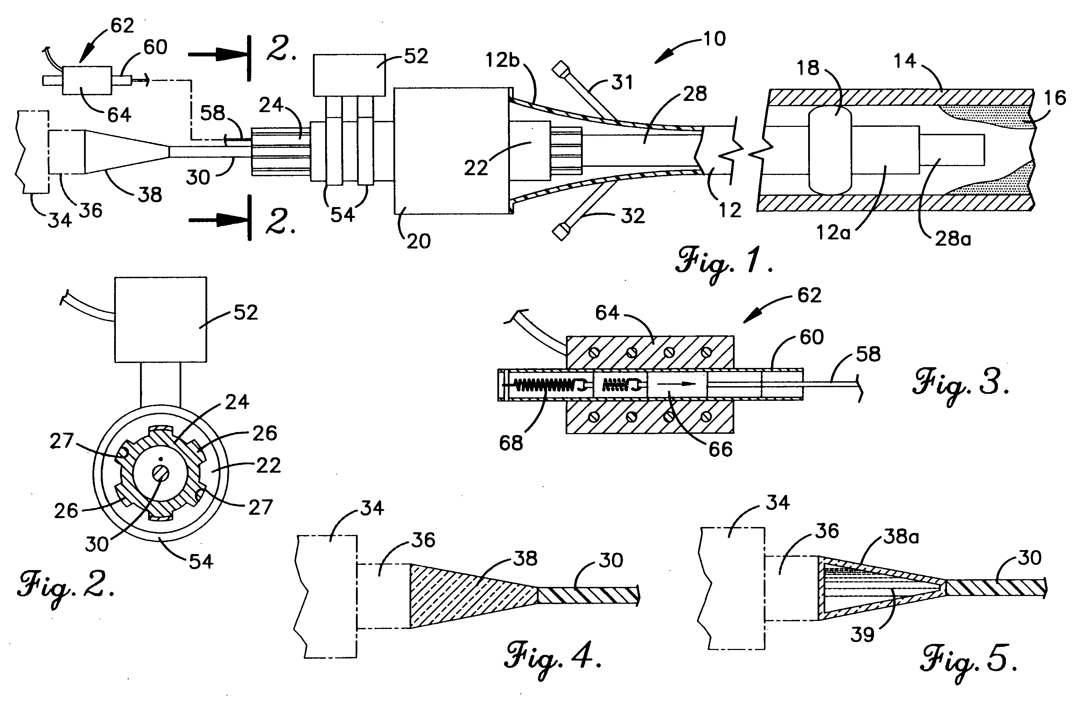

Referring now to the drawings in more detail and initially to Fig. 1, numeral 10 generally designates a catheter which is constructed according to a first embodiment of the present invention. The catheter 10 includes a hollow catheter tube 12 having an elongated configuration and a circular cross section. The catheter tube 12 has a size and construction to be inserted into the body to the area which is to undergo treatment. For example, if the catheter tube 12 is to be used for the treatment of atherosclerotic plaque, it should be small enough to be inserted into an artery such as the artery 14 which is afflicted by the presence of plaque 16 on the interior of the artery wall. An annular balloon seal 18 may be provided on the inner end 12a of the catheter tube in order to provide a seal against the artery wall.

The opposite or outer end of the catheter tube 12 is designated by numeral 12b in Fig. 1 and may be secured to a stationary motor 20 which is preferably an electrical stepping motor. Referring additionally to Fig. 2, the motor 20 rotates an output shaft 22. A sleeve 24 fits within the output shaft 22 of the motor and is provided with a plurality of splines 26 that interfit with internal key ways 27 on the shaft 22. Consequently, the sleeve 24 is rotated directly with the shaft 22 but is able to slide axially relative to shaft 22 by reason of the sliding movement that is permitted of the splines 26 within the key ways 27 which receive them.

Sleeve 24 is fitted on and rigidly fixed to an elongated tubular probe 28 which extends through the length of the catheter tube 12 and is coaxial with it. The probe 28 is smaller in diameter than the catheter tube 12 and has an outer end to which sleeve 24 is fixed and an inner end 28a which projects out of the inner end 12a of the catheter tube. An optical fiber 30 extends through substantially the entire length of the probe 28, with the free end of the fiber 30 terminating within the inner end 28a of the probe. The catheter tube 12 is equipped with lumens 31 and 32 for applying suction and/or fluid for irrigating or flushing the artery 14 or other area of the body into which the catheter is inserted.

A conventional ultrasonic transducer 34 is located externally of the catheter and outside of the body when the catheter is in use. The transducer 34 may be excited electrically in order to transmit ultrasonic signals via a piezo-electrode 36, and the transducer 34 also receives pulse echoes which are reflections of the transmitted signals. The received pulse echoes are transformed by the transducer into electrical signals to provide information as to the configuration, location and character of the tissues from which the signals are reflected. The transducer is preferably a piezo-electric ceramic crystal type of unit.

The piezo-electrode 36 is connected with the smaller optical fiber 30 by a tapered transition element 38 which may be a solid frusto-conical member constructed of a substance that conducts ultrasonic signals, as shown in Fig. 4. Alternatively, the transition element may be a hollow frusto-conical member 38a (see Fig. 5) filled with a fluid 39 which conducts ultrasonic signals. The large end of the transition element connects with the piezo-electrode 36, and its small end connects with the outer end of the optical fiber 30. The optical fiber 30 conducts the ultrasonic signals and transmits them to the catheter tip and back to the transducer as pulse echoes which reflect from the area at which the transmitted signals are directed. In this manner, the transducer and fiber 30 operate to provide an ultrasonic image of the area ahead of the catheter tip that is to be treated. The fiber 30 may be a quartz fiber coated with an external reflective material.

Electrical power is provided to the catheter 10 by an electrical transmitter or power source 52 through slip rings 54 mounted on the motor shaft 22.

As best shown in Figs. 6-8, the fiber 30 has a tip 30a located adjacent to the tip end 28a of probe 28. Mounted in the tip end 28a is a U-shaped sheath 56 having its bight portion located near the center of probe 28 and its opposite end located adjacent to the probe wall. The interior of the sheath 56 extends along a radius of the probe and closely receives the tip 30a of fiber 30. The opposite sides of the sheath 56 are provided with slots 56a which closely receive small pins 57a projecting from opposite sides of a collar 57 mounted rigidly on fiber 30. Each slot 56a inclines at a 45° angle toward the tip of probe 28 as it extends outwardly toward the probe wall. Consequently, as pins 57a move back and forth in slots 56a, the tip 30a of the fiber is moved radially from the center of probe 28 to its circumference.

An actuator wire 58 connects at one end with the collar 57 and acts to move the fiber tip. As best shown in Figs. 1 and 3, the opposite or outer end of the actuator wire 58 extends into a cylinder 60 forming part of a magnetic actuator 62 which adjusts the fiber tip 30a radially within probe 28. The cylinder 60 extends through an electro magnet 64 forming part of the actuator. Mounted slidably within cylinder 60 and connected with the wire 58 is a magnet 66 which is continuously urged to the right as viewed in Fig. 3 or toward the tip of the catheter assembly by a compression spring 68.

When the electromagnet 64 is deenergized, the spring 68 maintains the magnet 66 in the position shown in broken lines in Fig. 3. Then, the actuator wire 58 is fully extended and pushes the collar 57 outwardly to the position of Fig. 7. Because the pins 57a are retained in the slots 56a, the tip 30a is located adjacent the wall of probe 28 at its radially outermost position. However, when the electromagnet 64 is energized, the magnetic attraction it exerts on the magnet 66 causes the magnet to retract to the position shown in solid lines in Fig. 3, thus pulling wire 58 and moving the tip 30a radially inwardly to the position shown in Fig. 6. In this position, the tip 30a is near the center axis of probe 28. The fiber 30 has enough slack to allow it to bend as the tip 30a is reciprocated in this manner along the radius of the probe. Because the probe is rotatable and the fiber tip 30a is adjustable radially in the probe, the fiber tip is able to scan the entirety of the area ahead of the catheter tip in order to provide a comprehensive ultrasonic image of the area that is to be treated.

Fig 9 depicts an alternative arrangement in which the tips 30a of multiple fibers 30 are arranged in a radial row in the sheath 56. In this arrangement, the fibers are not radially adjustable. However, because the fiber tips 30a in the aggregate occupy a radius of the probe 28, they are able to scan the area ahead of the catheter when the probe is rotated. In this way, a comprehensive ultrasonic image is generated to provide information as to where the plaque or other abnormality is located and how it is shaped so that proper treatment can be effected.

As shown in Figs. 8 and 9, the entire area within the probe 28 outside of the sheath 56 is occupied by optical fibers 70. Laser energy is transmitted to the fibers 70 selectively from a conventional medical laser (not shown) to apply laser energy selectively from the tips of the fibers for treatment of the affliction. Alternatively, the treatment can be effected by an abrasive head 72 (Figs. 14 and 15) which essentially provides an end cap on the tip 28a of the probe. The abrasive head 72 removes tissue abnormalities by abrasive action when the probe is rotated with the head 72 held against the area that is being treated. The abrasive head 72 is provided with an aperture 74 that provides a window through which ultrasonic signals are transmitted from the single fiber tip 30a (in the case of Fig. 14) or multiple fiber tips (in the case of Fig. 15) and pulse echoes are reflected back to the fiber tips.

In operation of the catheter 10, the catheter tube 12 may be inserted into the artery 14 until the catheter tube end 12a is adjacent to the area of the plaque 16. the probe 28 may be rotated within the catheter tube, and rotation of the probe sweeps the fiber or fibers 30 around with it. The fiber may be adjusted radially as the probe is rotated to obtain complete information about the location and configuration of the plaque deposit. Alternatively, in the case of multiple fibers 30 arranged in a row on the radius of the probe (Figs. 9 and 15), the same information can be obtained without the need to radially adjust any of the fibers. In either case, the ultrasonic system provides a complete and accurate ultrasonic image of the plaque formation. This information is then used to control the laser such that the laser beams emitted from the optical fibers 70 are directed appropriately to destroy the plaque 16 while avoiding damage to the artery walls or other healthy tissue. The information can also be used to assure that the abrasive head 72 is properly applied for treatment of the afflicted area.

The ultrasonic transducer 34 is preferably operated in a pulse-echo mode and is controlled by a computer which controls other functions as well. The information provided by transducer 34 determines the thickness of the plaque deposit inwardly from the artery wall, its exact location and its configuration.

A graphic display of the output from the transducer may be provided. The display for transducer 34 can include the echo amplitude and time of flight information, and these may be incorporated into a graphic representation of the probe in the artery to show the distance ahead of the probe at which the plaque deposit is detected. The longitudinal and angular positions of the probe may be encoded and used to provide location data that is stored simultaneously with the ultrasonic data. When a probe position is repeated during the course of a particular procedure, the most recent data should overwrite the previous data in order to show how the procedure has changed the condition of the artery. The data may be transferred to memory storage at any time so that before and after comparisons can be later made.

Fig. 10 depicts an alternative construction of the tip end of the probe 28. In this arrangement, the probe 28 is stationary and receives a rotatable tube 76 which may be rotated in a manner similar to the probe in the arrangement previously described. The tip of the tube 76 is provided with an enlarged head 78 having a frusto-conical reflective surface 80 and a cylindrical surface 82 beyond the surface 80. The fiber 30 extends along the axis of tube 76 with its tip 30a turned radially outward within the head 78 and terminating at the surface 82 to direct ultrasonic signals outwardly in a radial direction. Consequently, when the tube 76 is rotated, the tip 30a sweeps the entire circumference of the artery wall to provide an ultrasonic image of the artery wall profile.

The additional optical fibers 70 which apply laser energy for treating the afflicted area are arranged in the wall of probe 28 and are spaced apart circumferentially in a circular pattern, as best shown in Fig. 11. The fibers 70 direct the laser energy forwardly from the probe tip toward the reflective surface 80 which reflects the laser energy outwardly in a radial direction, as indicated by the directional arrows 100 in Fig. 10. By energizing the fibers 70 selectively, the area that requires treatment is effectively treated.

The catheter depicted in Figs. 10 and 11 operates in substantially the manner previously described, except that the ultrasonic signals are directed radially at all times, and the laser beam 100 is likewise directed radially for the treatment of plaque or other abnormalities.

Figs. 12 and 13 illustrate still another arrangement of components in the tip end of the probe 28. A reflective head 103 is mounted on the tip end of the probe and presents a frusto-conical reflective surface 103a. As best shown in Fig. 13, a plurality of optical fibers 30 for transmitting ultrasonic signals are extended in the cylindrical wall of probe 28 and are spaced apart circumferentially in a circular pattern. Each fiber 30 is surrounded by a cluster or bundle of additional optical fibers 70 which transmit laser energy. The fibers 30 and 70 direct the signals they emit toward the reflective surface 103a which reflects them radially outwardly as indicated by the directional arrows in Fig. 12.

In the embodiment of Figs. 12 and 13, the optical fibers 30 transmit and receive ultrasonic signals to provide an ultrasonic image of the area to be treated. The other fibers 70 are selectively energized to apply laser energy to the area which is indicated by the ultrasonic image as requiring treatment.

The ultrasonic transducer 34 may be operated in the pulse echo mode. Returning echoes are characterized by amplitude, time of flight and frequency. This information defines a sector of the artery within which the reflective tissue is located. Lower frequency operation produces a broader ultrasonic beam for impingement on a normal artery wall to produce reflections from relatively thin deposits. Increasing the operating frequency produces a narrower beam that produces reflections only from deposits that protrude farther inwardly from the artery wall. Thus, the highest frequency at which a reflection is received from a particular deposit indicates the thickness of the deposit or the extent of the artery blockage.

A potentially ambiguous response, such as a response from a deposit on the outside curvature at a bend in an artery, can be resolved by rotating the probe while sweeping through the frequency range of the transducer. The longitudinal and angular positions of the probe are controlled by encoded mechanical devices. The encoders are used to provide location data simultaneously with the ultrasonic data.

The ultrasonic signals should be processed by a receiver/amplifier which may be broad banded in order to cover the entire operating frequency and width. It can incorporate a series of high pass filters that are switched in and out as the transducer excitation frequency is switched. Alternatively, a series of narrow to medium band width filters can be used and switched in sequence with a series of discrete excitation frequencies.

Alternatively, the transducer may be constructed to have a very narrow band width. The transducer can be excited at its nominal natural frequency or at some multiple thereof. This provides a more powerful ultrasonic output than a broad band transducer, and it may be more suitable for relatively large arteries. However, because a more powerful output produces a longer decay time for the initial pulse, a narrow band width system is relatively insensitive to reflectors that are very close to the probe tip. This problem can be overcome by providing the ultrasonic device with separate transmitting and receiving elements which are electrically isolated from one another so that the receiving element does not receive the initial excitation impulse. The receiving element is thus able to respond to reflection that would otherwise be impossible to distinguish from aberrations in the excitation pulse.

It should be noted that with either a broad band width or narrow band width system, an acoustic lens can be added to the face of the transducer to either increase or decrease the amount of beam spread at a given operating frequency and/or to alter the angle of the central ray of the beam with respect to the axis of the artery.
Still another alternative embodiment of the catheter is depicted in Figs. 16-21a. Referring first to Figs. 16-17, a plurality of optical fibers 70 for transmitting laser energy extend from a shutter mechanism 134 and through the probe 28. The fibers 70 direct laser energy forwardly to treat the area immediately ahead of the catheter tip. The fibers 70 are arranged uniquely such that the fibers whose inner ends (right ends as viewed in Figs. 16-17) are closest to the longitudinal axis of the probe, have their outer ends located farthest from the center of the probe. Conversely, the fibers whose inner ends are located farthest from the center of the probe have their outer ends located closest to the center.

As shown in Figs. 21 and 21a, the shutter 134 has a plurality of pivotal shutter elements 138. When the shutter elements 138 are pivoted fully outwardly as shown in Fig. 21a, the shutter is fully open, and the outer ends of all of the fibers 70 are exposed through the shutter. When the elements are pivoted inwardly from the fully opened position, the shutter progressively closes and the shutter opening 140 becomes smaller such that the ends of only some of the fibers are exposed through the shutter opening. The laser energy is transmitted through the shutter opening and is applied to those fibers whose ends are exposed.

It is noted that as the shutter progressively closes, the fiber ends farthest from the center of the probe are progressively covered by the shutter, and the corresponding fibers have their inner ends closest to the center of the probe. Consequently, as the shutter closes, the laser energy is progressively transmitted only through those fibers whose inner ends are located closest to the perimeter of the probe 28. As a result, as the shutter is closed, the area within the artery closest to the artery wall is treated last.

The catheter depicted in Figs. 16-17 is otherwise constructed like the catheter shown in Fig. 1, except for the arrangement of the optical fibers 30 which conduct ultrasonic signals to and from the area undergoing treatment and the transducers which apply ultrasonic signals to the fibers 30 and receive the reflected echoes from the fibers 30. A sleeve 150 forms an extension of sleeve 24 and carries four slip rings 152 which are axially spaced apart. As best shown in Fig. 17, each slip ring 152 is provided with a plurality of slip ring electrical contacts 154. The contacts 154 are annular and are contacted by mating electrical contacts 156 mounted on stationary blocks 157. A conventional electrical transmitter/receiver 158 is connected with contacts 156 by wiring 160. The electrical connection between the stationary contacts 156 and the slip ring contacts 154 allows electrical current to pass between the stationary and rotational parts.

Also mounted on sleeve 150 are four axially spaced disks 162. Each disk 162 extends radially from sleeve 150 and is provided with six ultrasonic transducers 164 which preferably take the form of piezo-electric crystals. The transducers 164 are circumferentially spaced on each disk 162. Each transducer 164 is connected by wires 166 with two of the slip rings 154 to allow electrical signals to be applied to and received from the transducer. An optical fiber 30 is provided for each transducer 164 to transmit ultrasonic signals from the transducer to the tip of the probe and receive pulse echo signals for transmission back to the transducer. The fibers 30 extend from the transducers through openings 168 in the sleeve 150 and probe 28, with the openings 168 providing access for the fibers into the interior of the probe.

The openings 168 are located between adjacent pairs of the disks 162.

The arrangement of Figs. 16-21a allows the catheter to be equipped with multiple transducers for obtaining a larger quantity of information from the ultrasonic system. Although four disks 162 each with six transducers 164 have been shown and described, it is to be understood that virtually any desired number of disks and/or transducers can be provided. The simple slip ring system provides electrical connection between the stationary and rotating parts.

Fig. 22 depicts an arrangement similar to that of Figs. 16-21a, except that the disks 162 are eliminated and the transducers are mounted directly on the slip rings 152.

Figs. 23-26 depict different patterns in which the fibers 30 for the ultrasound and the fibers 70 for the laser energy may be arranged in the probe 28. In Fig. 23, the fibers 30 are shown arranged in a plurality of concentric circles 160 in the probe. The fibers 70 are arranged in a plurality of clusters or bundles 162 each containing seven of the fibers. The bundles 162 are arranged in plural circles 164 located between the circles 160.

In the arrangement of Fig. 24, the fibers 30 are arranged in a spiral 166 which spirals outwardly from the center of the probe 28 to the wall of the probe. The other fibers 70 are packed in the space that is left unoccupied by fibers 30.

Fig. 25 depicts an alternative fiber pattern in which fibers 30 are arranged in a plurality of different spirals 168, each of which spirals outwardly from the center of the probe and terminates at its wall. The other fibers 70 are packed in the spaces between the spirals 168.

In the probe shown in Fig. 26, fibers 30 are arranged in a grid configuration in a plurality of straight rows. Fibers 70 are packed in the space left unoccupied by fibers 70.

Figs. 27 and 28 depict a modified catheter tube 12 in which the probe 28 has an inner tip portion 28a which is separate from the rest of the probe. Optical fibers 180 extend through the probe 28 and its tip portion 28a to transmit ultrasonic signals and/or laser energy. The tips of the fibers 180 are fixed to the tip portion 28a of the probe. The probe 28 has an angled end surface 28b that abuts the adjacent end surface 28c of the probe tip portion 28a. Both the probe 28 and its tip portion 28a are rotatable, and the tip portion 28a can be rotated relative to the probe by rotating the bundle of fibers 180. Surface 28b occupies a plane that is oriented at approximately 30 degrees to a plane transverse to the longitudinal axis of the probe, or at 60 degrees to the longitudinal axis of the probe.

When the tip portion 28a is in the rotative position shown in Fig. 27, it forms a straight extension of probe 28. However, when tip portion 28a is rotated 180 degrees relative to the probe to the position shown in Fig. 28, the tip portion 28a is oriented at a bend angle of about 60 degrees relative to the probe. By rotating the tip portion between the extreme positions of Figs. 27 and 28, the tip portion can be oriented relative to the probe at any desired angle between 0 degrees and 60 degrees. The angle of surfaces 28b and 28c relative to the longitudinal axis of the probe can be different from the sixty degree angle suggested and can be virtually any desired angle.

The ability to adjust the probe tip angle allows the probe to acommodate bends and curves such as the curved area 14a of artery 14. The aim of the optical fibers 180 can also be varied as desired. As the probe tip portion 28a is rotated, the fibers 180 are pulled tautly, and the tension of the fibers holds the surfaces 28b and 28c against one another to assure that the tip portion 28a will angle as intended.

Fig. 29 depicts an arrangement which is similar to that of Figs. 27 and 28. The principal difference is that in the Fig. 29 embodiment, an elastic sleeve 182 is fitted around probe 28 and its tip portion 28a at the joint between them. The end of the sleeve 182 is provided with an inturned rib 184 which fits slidably in a groove 186 formed in the tip portion 28a. The sleeve 182 may be fixed to probe 28 and acts to hold tip portion 28a such that the surfaces 28b and 28c are always in abutting contact with one another.

Fig. 30 depicts a catheter in which the probe 28 is provided with a tip portion 28a which is flexible at least between the positions shown in solid lines and dashed lines in Fig. 30. In order to flex the tip portion 28a, a pair of wires 188 are extended through the probe 28 and into the probe tip portion 28a. The wires 188 are located at diametrically opposed positions in the probe. Each wire 188 has an outturned end 190 which is secured to the tip portion 28a.

By pulling on one of the wires 188 and pushing the other wire, the tip portion 28a can be flexed as desired. For example, if the lower wire 188 is pulled as indicated by the directional arrow 192 and the upper wire 188 is simultaneously pushed as indicated by the directional arrow 194, the tip portion 28a is flexed downwardly in a curved shape from the dashed line position of Fig. 30 to the solid line position. By manipulating the wires 188 oppositely (pulling on the upper wire and pushing on the lower wire), the tip portion 28a can be flexed upwardly. When the probe is rotated, the wires are oriented to flex the tip portion to the side or in any desired direction. The extent to which the tip portion 28a is flexed is controlled by the force with which the wires are pushed and pulled, so the flexure is easily controlled. By properly manipulating the wires and the rotational position of the probe, the probe can be passed through curved portions of arteries and other body parts.

Fig. 31 depicts a catheter construction in which the probe 28 is flexible and is provided on its tip with an enlarged head 196 having a spherical shape. The spherical surface of the head 196 acts against the inside surface of the wall of the artery 14 and moves along the curved portion 14a to cause flexure of the probe 28. The spherical head 196 is preferably slightly smaller in diameter than the artery 14 and may be larger in diameter tham the probe 28, although it need not be. The guiding action of the curved surface on the head 196 allows the probe to pass into and through the curved area 14a without gouging or otherwise damaging the arterial wall with sharp edges or other jagged surfaces.

Ultrasonic fibers 180 for transmiting ultrasonic signals and/or laser energy extend through the probe 28. The tips of the fibers 180 terminate at the surface of the head 196 and occupy the forward hemisphere of the head. The fibers are thus able to transmit ultrasonic signals and/or laser energy toward plaque deposits 16 located anywhere on the wall of the artery 14.

The catheter depicted in Fig. 32 differs from that of Fig. 31 only in that the tips of the fibers 180 occupy only one-fourth of the surface area of the head 196. The ultrasonic signals and/or laser energy is thus more concentrated and useful in treating plaque deposits 16 which are more pronounced on one side of the artery wall, as shown in Fig. 32. The probe 28 can be turned to turn the head 196 in order to allow the entire arterial wall to be swept by the fiber tips.

Although the various embodiments of the invention have been described in connection with the treatment of arterial plaque, it is understood that the catheter can be used in the laser treatment of other medical conditions. For example, tumors and other abnormalities can be treated with laser energy in the colon, prostate, esophagus and other organs and internal body parts. It should also be understood that the various ultrasound systems can be used alone as forward looking ultrasound schemes for detecting the configurations in arteries and other internal body parts, as well as in combinations with other interactive treatment means such as atherectomy.

From the foregoing, it will be seen that this invention is one well adapted to attain all the ends and objects hereinabove set forth together with other advantages which are obvious and which are inherent to the structure.

It will be understood that certain features and subcombinations are of utility and may be employed without reference to other features and subcombinations. This is contemplated by and is within the scope of the claims.

Since many possible embodiments may be made of the invention without departing from the scope thereof, it is to be understood that all matter herein set forth or shown in the accompanying drawings is to be interpreted as illustrative and not in a limiting sense.

## Claims

1. A catheter (10) for insertion into the body to effect medical treatment comprising:
a catheter tube (12) and an elongated tubular probe (28) adapted for insertion into said catheter tube, said probe having a tip portion (28a) which is distinct from the remainder of the probe, said tip portion (28a) being flexible into one of a plurality of curved shapes relative to the remainder of said probe; and
a pair of wires (188) extending in said probe at diametrically opposed locations, each wire having a tip (190) fixed to said tip portion (28a) of said probe, said pair of wires being suitable for one wire being pulled and the other wire being simultaneously pushed to effect flexing of said tip portion into said one of a plurality of curved shapes relative to the remainder of said probe, for accommodating passage of the probe through a curve.

2. A catheter in accordance with Claim 1 further comprising a plurality of optical fibers (30,70,180) extending through said probe.

3. A catheter in accordance with Claim 2 wherein said plurality of optical fibers (30,70,180) are fixed to said tip portion (28a) of said probe (28).

4. A catheter in accordance with Claim 2 wherein said fibers (30,70,180) are configured to transmit at least one of ultrasonic signals and laser energy.

5. A catheter in accordance with Claim 2 wherein said fibers (30,70,180) are arranged within said tip portion (28a) in a defined pattern, said defined pattern comprising a geometric configuration for localizing the transmission of energy signals through said plurality of fibers.

6. A catheter in accordance with Claim 5 wherein said fibers (30) are arranged in a plurality of concentric circles (160) defined pattern.

7. A catheter in accordance with Claim 5 wherein said fibers (70) are arranged in a plurality of bundles (162) defined pattern, wherein each said bundle comprises a plurality of fibers.

8. A catheter in accordance with Claim 5 wherein said fibers (30) are arranged in a spiral (166) defined pattern extending from a center to an outer wall of said probe.

9. A catheter in accordance with Claim 1 wherein said elongated catheter tube (12) has a first end (12a) and a second end (12b), wherein said probe (28) extends through said catheter tube (12).

10. A catheter in accordance with Claim 9 wherein said probe tip portion (28a) extends beyond said catheter tube first end (12a) into the body.

11. A catheter in accordance with Claim 9 wherein said probe (28) is configured to be axially rotatable within said catheter tube (12).

12. A catheter in accordance with Claim 9 further comprising at least one annular balloon seal (18) secured to said catheter tube (12).

13. A catheter in accordance with Claim 2 further comprising at least one ultrasonic transducer (34) coupled to at least one of said optical fibers (30) to apply ultrasonic signals thereto for transmission along said fiber to said tip portion (28a) thereof and from said tip portion into the body, said transducer being operable to receive ultrasonic signals received from said tip portion and transmitted along said fibers.

## Patentansprüche

1. Katheter (10) zum Einführen in den Körper, zur Durchführung einer medizinischen Behandlung mit:
einem Katheterrohr (12) und einer länglichen rohrförmigen Sonde (28), die zum Einführen in das Katheterrohr angepaßt ist, wobei die Sonde einen Spitzenabschnitt (28a) hat, der sich vom Rest der Sonde unterscheidet, wobei der Spitzenabschnitt (28a) in eine von mehreren gekrümmten Formen relativ zum Rest der Sonde biegsam ist; und
einem Paar Drähten (188), die sich in der Sonde an diametral entgegengesetzten Stellen erstrecken, wobei jeder Draht eine am Spitzenabschnitt (28a) der Sonde befestigte Spitze (190) hat, wobei das Paar Drähte zum Ziehen von einem Draht und gleichzeitigen Schieben des anderen Drahts geeignet ist, um ein Biegen des Spitzenabschnitts in die eine von mehreren gekrümmten Formen relativ zum Rest der Sonde zu bewirken, um einen Durchgang der Sonde durch eine Krümmung zu ermöglichen.

2. Katheter nach Anspruch 1, ferner mit mehreren optischen Fasern (30, 70, 180), die sich durch die Sonde erstrekken.

3. Katheter nach Anspruch 2, wobei die mehreren optischen Fasern (30, 70, 180) am Spitzenabschnitt (28a) der Sonde (28) befestigt sind.

4. Katheter nach Anspruch 2, wobei die Fasern (30, 70, 180) so konfiguriert sind, daß sie Ultraschallsignale und/oder Laserenergie übertragen.

5. Katheter nach Anspruch 2, wobei die Fasern (30, 70, 180) innerhalb des Spitzenabschnitts (28a) in einem definierten Muster angeordnet sind, wobei das definierte Muster eine geometrische Konfiguration zum Lokalisieren der Übertragung von Energiesignalen durch die mehreren Fasern aufweist.

6. Katheter nach Anspruch 5, wobei die Fasern (30) in einem definierten Muster aus mehreren konzentrischen Kreisen (160) angeordnet sind.

7. Katheter nach Anspruch 5, wobei die Fasern (70) in einem definierten Muster aus mehreren Bündeln (162) angeordnet sind, wobei jedes Bündel mehrere Fasern aufweist.

8. Katheter nach Anspruch 5, wobei die Fasern (30) in einem definierten Spiralmuster (166) angeordnet sind, das sich von einer Mitte zu einer Außenwand der Sonde erstreckt.

9. Katheter nach Anspruch 1, wobei das längliche Katheterrohr (12) ein erstes Ende (12a) und ein zweites Ende (12b) hat, wobei sich die Sonde (28) durch das Katheterrohr (12) erstreckt.

10. Katheter nach Anspruch 9, wobei sich der Spitzenabschnitt (28a) der Sonde über das erste Ende (12a) des Katheterrohrs hinaus in den Körper erstreckt.

11. Katheter nach Anspruch 9, wobei die Sonde (28) so konfiguriert ist, daß sie innerhalb des Katheterrohrs (12) axial drehbar ist.

12. Katheter nach Anspruch 9, ferner mit mindestens einer ringförmigen Ballondichtung (18), die am Katheterrohr (12) befestigt ist.

13. Katheter nach Anspruch 2, ferner mit mindestens einem Ultraschall-Transducer (34), der mit mindestens einer der optischen Fasern (30) gekoppelt ist, um an ihr Ultraschallsignale zur Übertragung entlang der Faser zu ihrem Spitzenabschnitt (28a) und aus dem Spitzenabschnitt in den Körper anzulegen, wobei der Transducer so betriebsfähig ist, daß er Ultraschallsignale empfängt, die vom Spitzenabschnitt empfangen und entlang der Fasern übertragen werden.

## Revendications

1. Cathéter (10) à insérer dans le corps pour effectuer un traitement médical, comprenant :
un tube cathéter (12) et une sonde tubulaire allongée (28) adaptée pour être insérée dans ledit tube cathéter, ladite sonde étant munie d'une portion en pointe (28a) distincte du reste de la sonde, ladite portion en pointe (28a) étant flexible dans une forme parmi une pluralité de formes courbées par rapport au reste de ladite sonde ; et
une paire de câbles (188) s'étendant dans ladite sonde à des emplacements diamétralement opposés, chaque câble étant muni d'une pointe (190) fixée à ladite portion en pointe (28a) de ladite sonde, ladite paire de câbles étant adaptée pour un câble étant tiré et l'autre câble étant poussé simultanément pour effectuer un fléchissement de ladite portion en pointe dans ladite forme parmi une pluralité de formes courbées par rapport au reste de ladite sonde, pour adapter le passage de la sonde au travers d'une courbe.

2. Cathéter selon la revendication 1, comprenant en outre une pluralité de fibres optiques (30, 70, 180) s'étendant à travers ladite sonde.

3. Cathéter selon la revendication 2, dans lequel ladite pluralité de fibres optiques (30, 70, 180) est fixée à ladite portion en pointe (28a) de ladite sonde (28).

4. Cathéter selon la revendication 2, dans lequel lesdites fibres (30, 70, 180) sont configurées pour transmettre des signaux ultrasons et/ou de l'énergie laser.

5. Cathéter selon la revendication 2, dans lequel lesdites fibres (30, 70, 180) sont disposées en ladite portion en pointe (28a) dans un motif défini, ledit motif défini comprenant une configuration géométrique pour localiser la transmission des signaux d'énergie au travers de ladite pluralité de fibres.

6. Cathéter selon la revendication 5, dans lequel lesdites fibres (30) sont disposées en un motif défini en une pluralité de cercles concentriques (160).

7. Cathéter selon la revendication 5, dans lequel lesdites fibres (70) sont disposées en un motif défini en une pluralité de faisceaux (162), dans lequel chaque dit faisceau comprend une pluralité de fibres.

8. Cathéter selon la revendication 5, dans lequel lesdites fibres (30) sont disposées en un motif défini en spirale (166) s'étendant d'un centre à une paroi extérieure de ladite sonde.

9. Cathéter selon la revendication 1, dans lequel ledit tube cathéter allongé (12) est muni d'une première extrémité (12a) et d'une seconde extrémité (12b), dans lequel ladite sonde (28) s'étend à travers ledit tube cathéter (12).

10. Cathéter selon la revendication 9, dans lequel ladite portion en pointe de la sonde (28a) s'étend au-delà de ladite première extrémité du tube cathéter (12a) dans le corps.

11. Cathéter selon la revendication 9, dans lequel ladite sonde (28) est configurée pour être rotative de manière axiale à l'intérieur dudit tube cathéter (12).

12. Cathéter selon la revendication 9, comprenant en outre au moins un joint ballon annulaire (18) fixé audit tube cathéter (12).

13. Cathéter selon la revendication 2, comprenant en outre au moins un transducteur à ultrasons (34) couplé à au moins l'une desdites fibres optiques (30) pour y appliquer les signaux ultrasons pour la transmission le long de ladite fibre dans ladite portion en pointe (28a) et de ladite portion en pointe dans le corps, ledit transducteur étant apte à recevoir des signaux ultrasons reçus de ladite portion en pointe et transmis le long desdites fibres.
